# EUROPEAN PATENT SPECIFICATION

(11) **EP 1 695 954 B1**
(45) Date of publication and mention of the grant of the patent: **18.09.2013**
(21) Application number: 04793320.5
(22) Date of filing: 27.10.2004
(51) Int. Cl.: C07C 45/33, C07C 45/35, C07C 47/22, C07C 51/235, C07C 57/05, C07C 57/055, C07B 61/00

(54) **METHOD FOR PRODUCING (METH)ACRYLIC ACID OR (METH)ACROLEIN**
VERFAHREN ZUR HERSTELLUNG VON (METH)ACRYLSÄURE ODER (METH)ACROLEIN
PROCEDE DE FABRICATION D'ACIDE (METH)ACRYLIQUE OU DE (METH)ACROLEINE

(30) Priority: 15.12.2003 JP 2003416718
(43) Date of publication of application: 30.08.2006
(73) Proprietor: Mitsubishi Chemical Corporation, Chiyoda-ku Tokyo 100-8251 (JP)
(72) Inventor: YADA, Shuhei, Yokkaichi-shi, Mie 5108530 (JP); OGAWA, Yasushi, Yokkaichi-shi, Mie 5108530 (JP); SUZUKI, Yoshiro, Yokkaichi-shi, Mie 5108530 (JP); JINNO, Kimikatsu, Yokkaichi-shi, Mie 5108530 (JP)
(74) Representative: Vossius & Partner
(86) International application number: PCT/JP2004/016287
(87) International publication number: WO 2005/058785

(56) References cited:
- EP-A1- 0 987 057
- WO-A-03/076373
- JP-A- 08 092 154
- JP-A- 2003 252 820
- JP-A- 2003 306 457
- JP-B2- 53 030 688
- US-A- 5 821 390

## Description

### Technical Field

The present invention relates to a process for the production of acrolein or acrylic acid efficiently in a stable manner by a gas-phase catalytic oxidation of propylene using molecular oxygen.

### Background Art

(Meth)acrolein or (meth)acrylic acid is usually produced by a gas-phase catalytic oxidation reaction of propylene, propane, isobutylene or (meth)acrolein using molecular oxygen or gas which contains molecular oxygen in the presence of a compounded oxide catalyst using a multi-tubular reactor.

In a catalyst layer of a multi-tubular reactor used for a gas-phase catalytic oxidation reaction, it is often that hot spots (abnormally heat-generated points in the catalyst layer) are formed and many methods for suppressing the formation of such hot spots have been proposed.

In JP-A-7-10802, there is proposed a process for the production of (meth)acrolein or (meth)acrylic acid by a catalytic gas-phase oxidation reaction of a raw material for (meth)acrolein or (meth)acrylic acid with molecular oxygen or gas which contains molecular oxygen using a fixed bed multi-tubular reactor in which, in order to prevent the generation of hot spots (abnormal heat generating point in catalytic layer) which lower the yield of the aimed product, catalysts are successively filled in such as manner that a carried rate of the catalytically active substance becomes bigger from the inlet area for the raw material toward the outlet area.

In JP-A-8-92147, there is disclosed a method where a flow direction of fluid for removal of heat (hereinafter, referred to as "heat medium") in a reactor shell and a flow direction of reaction gas introduced into a reactor are made parallel and then flow of the heat medium is risen by meandering using a baffle plate to make the temperature difference of the heat medium between inlet and outlet of the reactor not more than 2-10°C whereby temperature of the heat medium is made uniform.

In JP-A-2000-93784, there is proposed a method in which flow of the raw material gas for the reaction and that of the heat medium are made parallel in a downward direction and stoppage of gas where the heat medium does not exist is prevented whereby formation of hot spots is suppressed. The method is also a method in which the raw material gas is supplied from the upper area of the reactor so that the catalyst layer in the reaction tube is made passed downward whereby only the catalyst near the inlet of the catalyst layer which is most easily deteriorated is made exchangeable.

In JP-B-53-30688, there is disclosed a method in the production of acrylic acid by oxidation reaction of propylene using an oxidation catalyst in which the catalyst at the area where hot spots are apt to be generated in an inlet for raw material gas is diluted with an inert substance.

In JP-A-51-127013, there is proposed a process for the production of propylene or isobutylene in a fixed bed reactor in the presence of an oxidation catalyst where a catalyst of a carried type and a catalyst of a molded type comprising substantially the same composition are combined.

In JP-A-3-294239, there is proposed a process for the production of acrolein and acrylic acid by a gas-phase catalytic oxidation of propylene using a fixed-bed multi-tubular reactor, characterized in that, plural kinds of catalysts having different activities which are prepared by modification of type and/or amount of alkaline earth metal element group which is a catalyst component are filled in such a manner that the activity becomes higher from inlet to outlet for the raw material gas toward outlet.

On the other hand, a multi-tubular reactor is charged with a solid catalyst inside and is used for the reaction of bringing the catalyst into contact with a raw material. The multi-tubular reactor is often used when reaction temperature is controlled by an efficient removal of big heat of reaction generated by a gas-phase catalytic oxidation reaction in which a substance to be oxidized is contacted to molecular oxygen in the presence of a solid catalyst and there is a necessity for the prevention of quick deterioration of the catalyst being exposed to an excessively high temperature (hot spots) by heat of reaction.

In addition, in a multi-tubular reactor, many tubes are usually placed in a vertical direction and, therefore, when a process fluid is flown from the upper area or the lower area, the process fluid flow side is made upflow or downflow. With regard to a heat medium, it is also able to be supplied from the upper area or the lower area to the reactor shell side.

Accordingly, similar to the common shell-and-tube heat exchanger, there are a parallel current type where flowing directions of the process fluid and the heat medium are same and a counter-current type where flowing directions of the process fluid and the heat medium are opposite. When the direction of flowing the fluid is also taken into consideration, there are 1) a parallel current type where process fluid is downflow while heat medium is downflow, 2) a counter-current type where process fluid is upflow while heat medium is upflow, 3) a counter-current type where process fluid is upflow while heat medium is downflow and 4) a counter-current type where process fluid is downflow while heat medium is upflow.

In such a multi-tubular reactor, there is adopted a method in which, when a heat medium is circulated in the outer side (shell side) of the reactor bundle, temperature necessary for the reaction is maintained and, at the same time, the same as in the case of heat exchanger frequently used in chemical plants, heat exchange is simultaneously conducted between a process fluid (process gas in the case of a gas-phase catalytic oxidation reaction) and a heat medium whereby deterioration or inactivation of the catalyst in the tube due to too much rise of the temperature of the process fluid (formation of hot spots) is prevented. However, in spite of the fact that the above-mentioned many inventions are proposed in a process for the production of (meth)acrolein or (meth)acrylic acid by a gas-phase catalytic oxidation reaction of propane, propylene or isobutylene using molecular oxygen or gas which contains molecular oxygen in the presence of a compounded oxide catalyst, heat of reaction in the gas-phase catalytic oxidation reaction is so high that, for example, when reaction temperature is raised for enhancing the production speed, temperature of specific position of the catalyst layer becomes too high resulting deterioration of the catalyst or a runaway reaction is resulted due to the temperature becomes higher than the allowable one for the catalyst whereupon there is caused a problem such as that the catalyst is no longer able to be used.

WO 2003/076373 shows a process for catalytic vapor phase oxidation of propylene or isobutylene to (meth) acrylic acid, wherein the temperature of the catalyst is measured at plural points.

### Disclosure of the Invention

An object of the present invention is to provide a process for the production of acrolein or acrylic acid by conducting a gas-phase catalytic oxidation reaction of a raw material for the production of acrolein or acrylic acid with molecular oxygen or gas which contains molecular oxygen using a multi-tubular reactor having plural tubular reactors equipped with one or more catalyst layer(s) in a direction of a tube axis in which, even after changing the reaction condition for raising the reaction temperature for enhancing the production speed, a quick rise in temperature is suppressed and inactivation of the catalyst is prevented whereby the production is carried out efficiently in a stable manner.

The present inventors have found that, in a plant where (meth)acrolein, (meth)acrylic acid, etc. are produced by a gas-phase catalytic oxidation of propane, isobutylene or propylene using a multi-tubular reactor, changes in temperature for heat medium is necessary when production speed is changed by changing the supplying amount of a raw material propylene for example, that a method for changing the temperature of the heat medium is a very important factor and that the method has a big influence on the state of reaction of the reactor thereafter whereupon the present invention has been accomplished.

Thus, in accordance with the present invention, there is provided a process for the production of acrylic acid or acrolein having the following constitutions whereby the above-mentioned object of the present invention is achieved.
1. A process for the production of acrylic acid or acrolein by a gas-phase catalytic oxidation reaction of propylene with molecular oxygen or gas which contains molecular oxygen using a multi-tubular reactor having such a structure that there are plural reaction tubes equipped with one or more Mo-Bi type catalyst layer(s) in a direction of tube axis and a heat medium for the adjustment of reaction temperature is able to flow outside those reaction tubes, wherein the process is characterized in that the change for raising the reaction temperature of said gas-phase catalytic oxidation reaction is conducted by means of change in inlet temperature of the heat medium for the adjustment of reaction temperature together with (1) change in the inlet temperature of the heat medium for the adjustment of reaction temperature is conducted at not higher than 2°C for each changing operation as such and (2) when a changing operation is continuously conducted, the changing operation is conducted where the time interval from the changing operation immediately before that is made not shorter than 10 minutes.
2. The process according to the above 1, wherein the difference between the maximum value of the reaction peak temperature of the catalyst layer of the reaction tube and the inlet temperature of the heat medium for adjustment of the reaction temperature is not lower than 20°C.
3. The process according to the above 1 or 2, wherein activity of each catalyst layer of the reaction tube is adjusted by mixing of an inert substance.
4. The process according to any of the above 1 to 3, wherein the number of the catalyst layer of the reaction tube is from 1 to 10.

### Brief Description of the Drawings

Fig. 1 is an approximate cross-sectional view which shows one embodiment of a multi-tubular reactor of a heat exchange type used for the gas-phase catalytic oxidation method of the present invention.
Fig. 2 is an approximate drawing which shows an embodiment of a baffle plate used for the multi-tubular reactor of a heat exchange type according to the present invention.
Fig. 3 is an approximate drawing which shows an embodiment of a baffle plate used for the multi-tubular reactor of a heat exchange type according to the present invention.
Fig. 4 is an approximate cross-sectional view which shows an embodiment of a multi-tubular reactor of a heat exchange type used for the gas-phase catalytic oxidation method of the present invention.
Fig. 5 is an enlarged approximate cross-sectional view of an intermediate tube plate which divides the shell of the multi-tubular reactor of a heat exchange type of Fig. 4.

With regard to the symbols in the drawings, 1b and 1c are reaction tubes; 2 is a reactor; 3a and 3b are circular introduction tubes; 3a' and 3b' are circular introduction tubes; 4a is a discharge opening for the product; 4b is a supplying inlet for the raw material; 5a and 5b are tube plates; 6a and 6b are perforated baffle plates; 6a' and 6b' are perforated baffle plates; 7 is a circulation pump; 8a and 8a' are supplying lines for heat medium; 8b and 8b' are pulling-out lines for heat medium; 9 is an intermediate tube plate; 10 is a heat shielding plate; 11, 14 and 15 are thermometers; 12 is a stagnant space; and 13 is a spacer rod.

### Best Mode for Carrying Out the Invention

The present invention will now be further illustrated as hereunder.

The present invention is characterized in that, in a gas-phase catalytic oxidation method for the production of acrylic acid or acrolein, condition for changing the temperature of a multi-tubular reactor in which a catalyst conducting the gas-phase catalytic reduction is filled is carried out by controlling the inlet temperature of a heat medium.

### (Reaction system)

Representative examples of the reaction system in the process for the production of acrolein and acrylic acid applied in industry are a one-pass system, a system where unreacted propylene is recycled and a system where waste gas after burning is recycled which will be mentioned below and, in the present invention, there is no limitation for the reaction system including those three ones.

### (1) One-pass system

This system is a method where, in a reaction of the former stage, propylene, air and steam are mixed, supplied and converted mainly into acrolein and acrylic acid and the outlet gas thereof is not separated from the product but is supplied to the reaction of the latter stage. A method in which air and steam which are necessary for the reaction in the reaction of the latter stage are supplied to the reaction of the latter stage in addition to the outlet gas in the reaction of the former stage at that time is also common.

### (2) System where unreacted propylene is recycled

This system is a method where the reaction production gas containing acrylic acid obtained in the latter stage reaction is introduced into a device for the collection of acrylic acid so that acrylic acid is collected as an aqueous solution while a part of waste gas containing unreacted propylene at the side of the collecting device for acrylic acid is supplied to the former stage reaction whereupon a part of the unreacted propylene is recycled.

### (3) System where waste gas after burning is recycled

This system is a method where the reaction production gas containing acrylic acid obtained in the latter stage reaction is introduced into a device for the collection of acrylic acid so that acrylic acid is collected as an aqueous solution, all of the waste gas at the side of the collecting device for acrylic acid is burned and oxidized, unreacted propylene, etc. contained therein are mainly converted into carbon dioxide and water and a part of the resulting burned waste gas is added to the former stage reaction.

A multi-tubular reactor is generally used in such a case that heat of reaction is very high as in the oxidation reaction whereby catalyst is to be protected by a strict control of the reaction temperature of the catalyst and property of the catalyst is to be highly maintained so that productivity of the reactor is to be enhanced.

In recent years, production amounts of acrylic acid from propylene and methacrylic acid from isobutylene (both acrylic acid and methacrylic acid will be referred to as (meth)acrylic acid) have significantly increased as a result of an increase in the demand therefor. Thus, many plants have been constructed in the world and production scale of the plant has been expanded to not less than 100,000 tons per year for each plant. As the production scale of the plant increases, it is necessary to increase the production amount for each oxidation reactor and, as a result, load of a gas-phase catalytic oxidation reactor for propane, propylene or isobutylene is becoming large. As a result thereof, there has been a demand for highly efficient multi-tubular reactors.

In the present invention, there is adopted a system where a substance to be oxidized (i.e., propylene) is subjected to a gas-phase catalytic oxidation by gas which contains molecular oxygen using a multi-tubular reactor in which a cylindrical reactor shell having a raw material supplying inlet and a product discharging outlet, plural circular introduction tubes for introducing a heat medium into the cylindrical reactor shell or taking out the same therefrom which are set at the outer circumference of the cylindrical reactor shell, a circulation device where the plural circular introduction tubes are connected each other, plural reaction tubes which are arrested by plural tube plates of the reactor and contains the catalyst and plural baffle plates for changing the direction of the heat medium introduced into the reactor shell are installed in the longitudinal direction of the reactor. In the above reaction tube, an oxidation catalyst, i.e., an Mo-Bi type catalyst and optionally an Mo-V type catalyst is filled. The characteristic of the process of the present invention is that, even in changing the temperature condition, a stable continuous operation is able to be carried out particularly owing to a due setting of temperature of the heat medium.

The present invention is a gas-phase catalytic oxidation method in which propylene optionally in admixture with propane, isobutylene or (meth)acrolein or a mixture thereof is used as a substance to be oxidized and subjected to a gas-phase catalytic oxidation using gas which contains molecular oxygen to prepare acrolein or acrylic acid. (Meth) acrolein, (meth)acrylic acid or both are prepared from propylene, propane and isobutylene. (Meth)acrylic acid is prepared from (meth)acrolein.

"Process gas" in the present invention means gas which participates in the gas-phase oxidation reaction such as a substance to be oxidized as a raw material gas, gas which contains molecular oxygen and the resulting product. "Raw material" means a substance to be oxidized.

### Composition of raw material gas

A mixed gas containing at least one substance to be oxidized, i.e., propylene and optionally propane, isobutylene or (meth)acrolein, as a raw material gas, gas which contains molecular oxygen and steam is mainly introduced into a multi-tubular reactor used for the gas-phase catalytic oxidation.

In the present invention, concentration of the substance to be oxidized in the raw material gas is 6 to 10 molar % while oxygen and steam are 1.5- to 2.5-fold mol and 0.8- to 5-fold mol, respectively, to the substance to be oxidized. The raw material gas introduced thereinto is divided into each reaction tube, passes through the inside of the reaction tube and reacts in the presence of the oxidation catalyst charged therein.

### Multi-tubular reactor

The gas-phase catalytic oxidation reaction using a multi-tubular reactor is a method which has been widely used for the production of (meth)acrylic acid or (meth)acrolein from at least one substance to be oxidized as defined above using molecular oxygen or molecular oxygen-containing gas in the presence of a compounded oxide catalyst.

The multi-tubular reactor used in the present invention has been commonly used in industry and there is no particular limitation therefor.

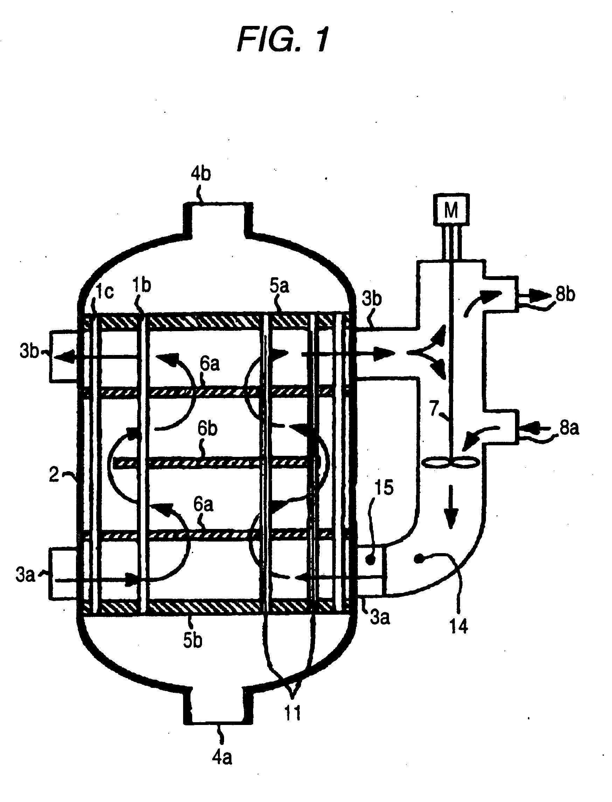

As hereunder, an embodiment of the present invention will be illustrated according to Fig. 1 to Fig. 5. Fig. 1

Fig. 1 is an approximate cross-sectional view which shows an embodiment of a multi-tubular reactor of a heat exchange type used for the gas-phase catalytic oxidation method of the present invention.

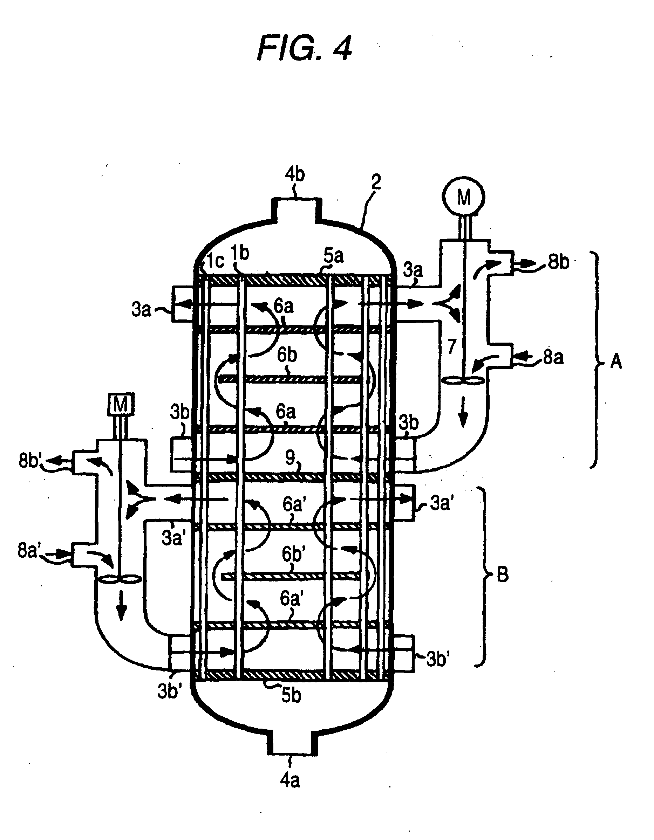

Reaction tubes 1b, 1c are fixed to tube plates 5a, 5b and set in a shell 2 of a multi-tubular reactor. A raw material supplying inlet which is an inlet for raw material gas for the reaction and a product discharging outlet which is an outlet for the product are 4a or 4b. When the flow of process gas and heat medium are in a counter-current manner, the flow direction of the process gas may be in any direction but, since the flow direction of the heat medium in the reactor shell is mentioned by an arrow as an upward stream in Fig. 1, 4b is a raw material supplying inlet. On the outer circumference of the reactor shell, a circular introduction tube 3a for introduction of the heat medium is set. The heat medium where the pressure is made high by a circulation pump 7 for the heat medium ascends in the reactor shell from the circular introduction tube 3a returns from the circular introduction tube 3b to the circulation pump as a result of conversion of the flow direction by mutual arrangement of each plural perforated baffle plates 6a having an opening near the center of the reactor shell and perforated baffle plates 6b arranged so as to have an opening between the outer circumference and the reactor shell. A part of the heat medium absorbing the heat of reaction is cooled by a heat exchanger (not shown in the drawing) from a exhaust tube installed at the upper part of the circulation pump 7 and is introduced again into the reactor from the heat medium-supplying line 8a. Adjustment of temperature of the heat medium is carried out by controlling the thermometer 14 by adjusting the temperature or the flow rate of the circulated heat medium introduced from the heat medium-supplying line 8a.

Although being dependent upon the property of the catalyst used, temperature adjustment of the heat medium is conducted in such a manner that the difference of temperature of the heat medium between the heat medium-supplying line 8a and the heat medium-discharging line 8b is made 1 to 10°C or, preferably, 2 to 6°C.

It is preferred to install a rectification plate (not shown in the drawing) in the trunk plate part of the inside of circular introduction tubes 3a and 3b so that distribution of the flow rate of the heat medium in the circumferential direction is made minimum. A porous plate, a plate having slits or the like is used as the rectification plate and rectification is carried out in such a manner that opening area and slit interval of the porous plate are changed so that the heat medium flows thereinto from the whole circumference at the same flow rate. Temperature in the circular introduction tube (3a; preferably, together with 3b) is able to be monitored by installation of plural thermometers 15.

Although there is no particular limitation for the number of baffle plates installed in the reactor shell, it is preferred to install three plates (two plates of a 6a type and one plate of a 6b type) as usual. As a result of the existence of the baffle plate, an upward flow of the heat medium is inhibited but converted to a transverse direction to the direction of tube axis of the reaction tube and the heat medium is collected to the center from the outer circumference of the reactor shell, converted its direction at the opening of the baffle plate 6a, comes to the outer circumference and reaches the outer cylinder of the shell. The heat medium is converted again in its direction at the outer circumference of the baffle plate 6b, collected to the center, ascends the opening of the baffle plate 6a, comes to the outer circumference along the upper tube plate 5a of the reactor shell, passes the tubular introduction tube 3b and circulated to a pump.

A thermometer 11 is inserted into a reaction tube arranged in the reactor so that signal is transmitted even to the outside of the reactor and temperature distribution of the catalyst layer in the direction of tube axis of the reactor is recorded. Plural thermometers are inserted into the reaction tube and a thermometer measures the temperatures of 5 to 20 points in the direction of tube axis.

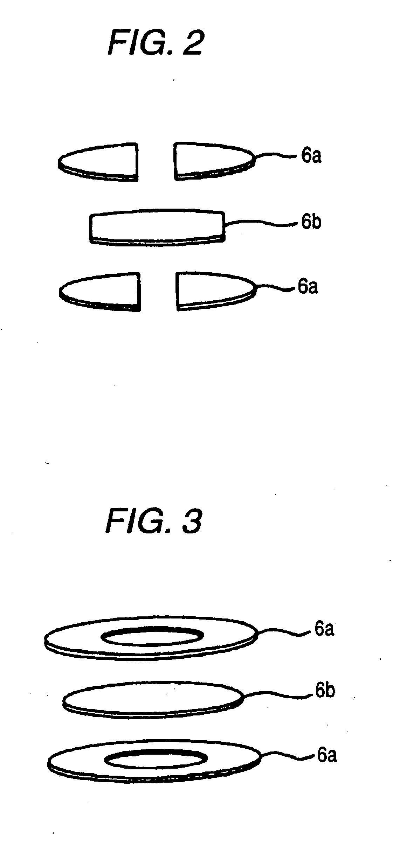

### Fig. 2, Fig. 3: Baffle plate

With regard to a baffle plate used in the present invention, any of an eclipsed circular baffle plate of a segment type as shown in Fig. 2 and a disk-type baffle plate as shown in Fig. 3 may be used so far as it is in such a constitution that the baffle plate 6a has an opening near the center of the reactor shell, the baffle plate 6b has an opening between the outer circumference and the outer cylinder of the shell and the heat medium changes its direction at each opening so that a by-pass flow of the heat medium is prevented and flow rate is able to be changed. In both types of baffle plates, the relation between the direction of flow of the heat medium and the tube axis of the reaction tube is unchanged.

With regard to a usual baffle plate, a baffle plate of a disk type as shown in Fig. 3 is often used. It is preferred that the central opening area of the baffle plate 6a is 5 to 50% or, more preferably, 10 to 30% of the cross-sectional area of the reactor shell. It is preferred that the opening area of the reactor shell trunk plate 2 of the baffle plate 6b is 5 to 50% or, more preferably, 10 to 30% of the cross-sectional area of the reactor shell. When the opening ratio of the baffle plates (6a and 6b) is too small, passage of the heat medium becomes long, pressure loss between the circular introduction tubes (3a and 3b) increases and power of the circulation pump 7 for the heat medium becomes big. When the opening ratio of the baffle plate is too large, numbers of the reactors (1c) increase.

Intervals of the baffle plates installed (interval between the baffle plates 6a and 6b; and interval between the baffle plate 6a and the tube plates 5a, 5b) are equal in many cases although it is not always necessary to make the intervals equal. It is recommended to set in such a manner that the necessary flow rate of the heat medium determined by heat of oxidation reaction generated in the reaction tube is ensured and that pressure loss of the heat medium becomes low. In the circular introduction tube 3a at the inlet for the heat medium, it should be avoided that the hot spot position of temperature distribution in the reaction tube and the position of the baffle plate become same. That is because, since the flow rate of the heat medium near the surface of the baffle plate lowers and coefficient of heat transfer is low, the hot spot temperature becomes far higher when the position to the hot spot is overlapped.

In order to avoid that the hot spot position and the baffle plate position become same, it is preferred to investigate in advance by means of experiments using a device of a small scale (such as bench equipment and pilot equipment) or by means of computer simulation.

### Fig. 4

Fig. 4 shows an approximate cross-sectional view of a multi-tubular reactor when the shell of the reactor is divided by an intermediate tube plate 9 and the gas-phase catalytic oxidation method of the present invention also includes a method using the same. In each of the divided spaces, different heat media are circulated and are controlled at different temperatures. The raw material gas may be introduced from any of 4a and 4b and, in Fig. 4, the flow direction of the heat medium in the reactor shell is shown by an arrow as an ascending flow and, therefore, 4b where the flow of the raw material gas process gas is in a counter-current direction to the flow of the heat medium is a supplying inlet for the raw material. The raw material gas introduced from the raw material supplying inlet 4b successively reacts in the reaction tube of the reactor.

In the multi-tubular reactor shown in Fig. 4, heat media in different temperatures are present in the upper and the lower areas (area A and area B in Fig. 4) in the reactor partitioned by the intermediate tube plate 9 and, accordingly, there are the following cases in the reaction tube. Thus, 1) a case where the same catalyst is filled in whole part and the reaction is carried out in which temperature is changed at the inlet and the outlet for raw material gas in the reaction tube; 2) a case where a catalyst is filled in the inlet part of the raw material gas and no catalyst is filled in the outlet part to make it hollow or an inert substance having no reactivity is charged so that the reaction product is quickly cooled; and 3) a case where different catalysts are filled in the inlet and the outlet parts for the raw material gas and, between them, no catalyst is filled in the outlet part to make it hollow or an inert substance having no reactivity is charged so that the reaction product is quickly cooled.

For example, propylene and optionally propane or isobutylene as a mixed gas with molecular oxygen-containing gas is introduced from a raw material supplying inlet 4b into a multi-tubular reactor shown in Fig. 4 to be used for the present invention and, firstly, it is made into (meth)acrolein in the first stage for the former stage reaction (area A of the reaction tube) followed by oxidizing said (meth)acrolein in the second stage for the latter stage reaction (area B of the reaction tube) to prepare (meth)acrylic acid. In the first stage part (hereinafter, may be referred to as "former stage part") and in the second stage part (hereinafter, may be referred to as "latter stage part"), different catalysts are filled and each of them is controlled at different temperatures whereby the reaction is carried out under the optimum condition. It is preferred that an inert substance which is not participated in the reaction is filled in the part where an intermediate tube plate exits between the former stage part and the latter stage part of the reaction tube.

### Fig. 5

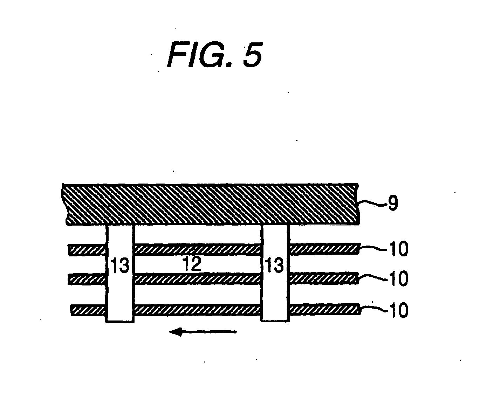

In Fig. 5, an intermediate tube plate is shown in an enlarged manner. The former and the latter stage parts are controlled at different temperatures and, when the difference in temperatures exceeds 100°C, heat transfer from the high-temperature heat medium to the low-temperature heat medium is no longer negligible and the precision of reaction temperature at the low-temperature side tends to deteriorate. In such a case, it is necessary to conduct heat insulation to prevent the heat transfer above or below the intermediate tube plate. Fig. 5 is the case where a heat insulation plate is used and it is preferred that two or three heat insulation plates 10 are placed at the position which is about 10 cm above or below the intermediate tube plate so that a stagnant space 12 where the heat medium is full without flow is formed whereby a heat insulation effect is achieved. The heat insulation plate 10 is fixed to the intermediate tube plate 9 by, for example, a spacer rod 13.

In Fig. 1 and Fig. 4, the direction of flow of the heat medium in the reactor shell is shown by an arrow as an ascending flow although a reverse direction is also possible in the present invention. In deciding the direction of the circulation flow of the heat medium, a phenomenon where gas or, to be more specific, inert gas such as nitrogen which may be present at the upper end of the circulation pump 7 and the reactor shell 2 is caught in a heat medium flow should be avoided. In case the heat medium is an ascending flow (Fig. 1), a cavitation phenomenon is noted in the circulation pump when gas is caught at the upper part in the circulation pump 7 whereby, in the worst case, the pump is destroyed. In case the heat medium is a descending flow, a catching phenomenon of gas takes place at the upper part of the reactor shell and a retained part of gas phase is noted at the upper part of the shell whereby the upper part of the reaction tube where said area where gas is retained is not cooled by the heat medium.

As a means for preventing the gas retention, it is essential that a gas-releasing line is formed whereby gas in the gas layer is substituted with the heat medium and, for such a purpose, the heat medium pressure in a heat medium-supplying line 8a is made high and a discharging line 8b for the heat medium is set at the place of as high as possible whereby a rise in the pressure in the shell is intended. It is preferred that the discharging line for the heat medium is located at least above the tube plate 5a.

When a multi-tubular reactor as shown in Fig. 1 is adopted in a multi-tubular reactor where propylene and optionally propane or isobutylene is oxidized with a molecular oxygen-containing gas and when the process gas is in a decending flow or, in other words, when the raw material gas comes from 4b and the product is discharged from 4a, concentration of (meth)acrolein which is an aimed product is high near the product discharging outlet 4a of the reactor and heating takes place by the heat of reaction whereby temperature of the process gas becomes high as well. Accordingly, in such a case, it is preferred that a heat exchanger is placed after the reactor 4a in Fig. 1 to well cool the process gas whereby an automatic oxidation reaction of (meth)acrolein does not take place.

When a multi-tubular reactor as shown in Fig. 4 is adopted and the process gas is a decending flow or, in other words, when the raw material gas comes from 4b and the product is discharged from 4a, concentration of (meth)acrolein which is an aimed product is high near the intermediate tube plate 9 which is an endpoint of the reaction of the first stage (area A of the reaction tube) and heating takes place by the heat of reaction whereby temperature of the process gas becomes high as well. Further, when the catalyst is filled only in the first stage (area A of the reaction tube: 5a-6a-6b-6a-9), the reaction is not carried out in the second stage of the reaction tubes 1b, 1c (area B of the reaction tube: between 9 and 5b) and the process gas is cooled by a heat medium flowing the passage of the shell side whereby (meth)acrolein is made in such a state that no automatic oxidation reaction takes place. In that case, catalyst is not filled in the area B (between 9 and 5b) of the reaction tubes 1b, 1c but the area is made hollow or solid having no reactivity is filled therein. The latter is preferred for making the characteristic of the heat transfer better.

When different catalysts are filled in the first stage (area A of the reaction tube: 5a-6a-6b-6a-9) of the multi-tubular reactor shown in Fig. 4 and in the second stage (area B of the reaction tube: 9-6a'-6b'-6a'-5b) of the same whereupon (meth)acrolein is prepared from propylene and optionally propane or isobutylene in the first stage while (meth)acrylic acid is prepared in the second stage, temperature of the catalyst layer of the first stage is high as compared with temperature of the catalyst layer of the second stage. To be more specific, temperatures of the areas near the endpoint of the reaction of the first stage (6a-9) and near the initiation point of the reaction of the second stage (9-6a') become high and, therefore, it is preferred that no reaction is carried out at those areas but the process gas is cooled by the heat medium flowing the passage at the shell side whereby (meth)acrolein is made in such a manner that no automatic oxidation reaction takes place. In that case, there is formed a part where catalyst is not filled in the area near the intermediate tube plate 9 (among 6a-9-6a' of the reaction tubes 1b, 1c) but the area is made hollow or solid having no reactivity is filled therein. The latter is preferred for making the characteristic of the heat transfer better.

### Hot spots

The raw material gas passing through a reaction tube is firstly heated when it passes through a lowly active catalyst layer filled in the raw material gas inlet part of the reaction tube and reaches the reaction initiation temperature. The raw material is oxidized by the catalyst filled as the next layer in the reaction tube and temperature further rises by the heat of oxidation reaction. Reacting amount is most abundant in the catalyst layer near the inlet for the raw material gas and, usually, heat of reaction which is much more generated than the removed heat amount by the heat medium acts for ascending the temperature of raw material gas whereupon hot spot is formed.

Although being dependent upon the adjustment of catalytic activity, there are many cases where hot spot is formed at the position of 10 to 80% of the full length of the reaction tube from the raw material gas inlet of the reaction tube. For example, when a reaction tube of 3 to 4 m is used, it is formed at the position of 0.3 to 3.2 m from the raw material gas inlet of the reaction tube.

When amount of the heat of reaction generated here exceeds the ability of removal of heat of the heat medium from outside of the reaction tube, temperature of the raw material gas rises more and more and generation of heat of reaction also increases. Finally, a runaway reaction is resulted exceeding the highest temperature by which the catalyst is resistant whereby the catalyst is changed in terms of its quality causing deterioration and destruction. When an illustration is made taking the case of a former stage reactor for the production of acrolein by oxidation reaction of propylene by a molecular oxygen-containing gas as an example, temperature of the heat medium is 250 to 350°C and the allowable highest temperature of the hot spot is 400 to 500°C. Further, temperature of the heat medium in the latter stage reactor for the production of acrylic acid by oxidation of acrolein by a molecular oxygen-containing gas is 200 to 300°C and the allowable highest temperature of the hot spot is 300 to 400°C.

### Diameter of reaction tube

Due to the fact that inside of the reaction tube enclosing the catalyst in an oxidation reactor is in a gas phase and that linear velocity of gas is restricted by resistance of the catalyst and coefficient of heat transfer in the tube is smallest resulting in rate-limiting by heat transfer, inner diameter which greatly affects the linear velocity of gas is very important.

Inner diameter of the reaction tube of the multi-tubular reactor according to the present invention is affected by heat quantity of reaction in the reaction tube and particle size of the catalyst and it is preferred to be 10 to 50 mm and, more preferably, 20 to 30 mm. When the inner diameter of the reaction tube is too small, many reaction tubes are necessary for filling the necessary total amount of the catalyst whereby labor for the manufacture of the reactor becomes big, much cost for the manufacture is needed and industrial economy becomes bad. On the other hand, when the inner diameter of the reaction tube is too big, surface area of the reaction tube for the necessary amount of the catalyst becomes small whereby area of heat transfer for removal of heat of reaction becomes small.

### Heat medium and coefficient of heat transfer

With regard to a heat medium flowing at the shell side of the reactor, a niter which is a mixture of nitrates is often used although a heat medium of a phenyl ether type which is an organic liquid type may be used as well.

As a result of flow of the heat medium, heat of reaction in the reaction tube is removed and it has been found that, in the heat medium introduced into the reactor shell from a circular introduction tube 3a for introduction of the heat medium, there are a region where it flows to the center from the outer circumference of the reactor and a region where a direction of flow is reversed at the center and that, in each region, effect of removal of heat is extremely different. When the direction of flow of the heat medium is at right angles to the tube axis of the reaction tube, coefficient of heat transfer is 1,000 to 2,000 W/m²°C while, when it is not at right angles, although being different whether the flow rate is upward or downward, it is 100 to 300 W/m²°C when a niter is used as a heat medium.

On the other hand, coefficient of heat transfer in the catalytic layer in the reaction tube of course depends upon the flow rate of the raw material gas but, since it is only about 100 W/m²°C, it goes without saying that rate-limitation of heat transfer is the gas phase in the tube the same as in the recognition up to now. Specifically, when flow of the heat medium is at right angles to the reaction tube axis, heat transfer resistance outside the tube is 1/10 to 1/20 of that of gas in the tube and, even when flow rate of the heat medium side changes, the overall influence on the heat transfer resistance is small. However, when the heat medium is in a flow of being parallel to the tube axis, coefficients of heat transfer outside and inside of the reaction tube are similar whereby the state of fluid outside the tube greatly affects the efficiency of removal of heat. Thus, when coefficient of heat transfer outside the tube is 100 W/m²°C; the overall coefficient of heat transfer is one half of that and, in addition, one half of the change in the heat transfer resistance outside the tube affects the overall coefficient of heat transfer.

### Catalyst

With regard to the catalyst used for a gas-phase catalytic oxidation reaction for the production of (meth) acrylic acid or (meth)acrolein, there are that which is used for the former stage reaction from olefin to unsaturated aldehyde or unsaturated acid and that which is used for the latter stage reaction from unsaturated aldehyde to unsaturated acid.

With regard to a compounded oxide catalyst of an Mo-Bi type used for the previous stage reaction (reaction from olefin to unsaturated aldehyde or unsaturated acid) for mainly producing acrolein by a gas-phase catalytic oxidation reaction, that which is represented by the following formula (I) may be listed.

MoₐW_{b}Bi_{c}Fe_{d}AₑB_{f}C_{g}DₕEᵢOₓ (I)

In the above formula (I), A is at least one element selected from nickel and cobalt; B is at least one element selected from sodium, potassium, rubidium, cesium and thallium; C is at least one element selected from alkaline earth metals; D is at least one element selected from phosphorus, tellurium, antimony, tin, cerium, lead, niobium, manganese, arsenic, boron and zinc; E is at least one element selected from silicon, aluminum, titanium and zirconium; and O is oxygen. Each of a, b, c, d, e, f, g, h, i and x means atomic ratio of Mo, W, Bi, Fe, A, B, C, D, E and O, respectively and, when a = 12, then 0 ≤ b ≤ 10, 0 < c ≤ 10 (preferably, 0.1 ≤ c ≤ 10), 0 < d ≤ 10 (preferably, 0.1 ≤ d ≤ 10), 2 ≤ e ≤ 15, 0 < f ≤ 10 (preferably, 0.001 ≤ f ≤ 10), 0 ≤ g ≤ 10, 0 ≤ h ≤ 4 and 0 ≤ i ≤ 30 and x is a value which is determined by the oxidized state of each element.

With regard to a compounded oxide catalyst of an Mo-V type used in the latter stage reaction (reaction from unsaturated aldehyde to unsaturated acid) for the production of acrylic acid by oxidation of acrolein in the above-mentioned gas-phase catalytic oxidation reaction, that which is represented by the following formula (II) may be listed.

MoₐV_{b}W_{c}Cu_{d}XₑY_{f}O_{g} (II)

In the above formula (II), X is at least one element selected from Mg, Ca, Sr and Ba; Y is at least one element selected from Ti, Zr, Ce, Cr, Mn, Fe, Co, Ni, Zn, Nb, Sn, Sb, Pb and Bi; and O is oxygen. Each of a, b, c, d, e, f and g is atomic ratio of Mo, V, W, Cu, X, Y and O, respectively and, when a = 12, then 2 ≤ b ≤ 14, 0 ≤ c ≤ 12, 0 < d ≤ 6, 0 ≤ e ≤ 3 and 0 ≤ f ≤ 3 and g is a value determined by the oxidized state of each element. The above-mentioned catalysts may be prepared by the method disclosed, for example, in JP-A-63-54942, JP-B-6-13096 and JP-B-6-38918.

The catalyst used in the present invention may be a molded catalyst which is molded by an extrusion molding method or a tablet compression method or may be a carried catalyst where a compounded oxide comprising catalytic components is carried on an inert carrier such as silicon carbide, alumina, zirconium oxide or titanium oxide.

There is no particular limitation for the shape of the catalyst used in the present invention but any of shapes in sphere, column, cylinder, star and ring and amorphous shape may be used.

### Diluent

The above-mentioned catalyst may be used by mixing with an inert substance as a diluent.

There is no particular limitation for the inert substance so far as it is stable under the above reaction condition and does not react with the raw material substance and the product. To be more specific, a substance used as a carrier for catalysts such as alumina, silicon carbide, silica, zirconia oxide or titanium oxide is preferred.

There is no particular limitation for its shape the same as in the case of the catalyst and any shape in sphere, column, cylinder, star, ring, small flake or net and amorphous shape may be used. Its size, etc. may be decided by taking diameter of the reaction tube and pressure loss into consideration.

Amount of the inert substance used as a diluent may be appropriately decided depending upon the aimed catalytic activity.

### Catalyst layer, adjustment of activity, etc.

Activity of a catalyst layer in the reaction tube may be changed.

With regard to a method for the adjustment of changing the activity of a catalyst layer in the reaction tube, there are a method where composition of the catalyst is adjusted and catalyst having different activity is used for each catalyst layer, a method where catalyst particles are mixed with particles of inert substance to dilute the catalyst whereby activity of each catalyst layer is adjusted, etc.

A specific example for the latter method is that the catalyst layer is made, for example, into two layers where a catalyst layer of the raw material gas inlet part of the reaction tube is made into a catalyst layer having a high ratio of inert substance particles in which the ratio of the inert substance particles used is made, for example, from 0.3 to 0.7 to the catalyst so that a low-activity layer is prepared while, in the catalyst layer at the outlet side of the reaction tube, the ratio is made, for example, as low as from 0 to 0.5 or a non-diluted catalyst is filled so that a high-activity layer is prepared.

There is no particular limitation for the numbers of the catalyst layer formed in the direction of tube axis of the multi-tubular reactor. However, when the numbers of the catalyst layers are too many, much labor is needed for the work of filling the catalyst and, therefore, numbers of the catalyst layer are usually from 1 to 10. With regard to the length of each catalyst layer, its optimum value is decided by catalyst species, catalyst layer number, reaction condition, etc. and, therefore, that may be appropriately decided so that the advantage of the present invention is able to be fully achieved.

### Reaction peak temperature

"Reaction peak temperature" is the maximum value of peak temperature for each catalyst layer of the reaction tubes which are present in plural numbers.

When the catalyst layer is in a multi-layered state, the "reaction peak temperature" which is compared with the inlet temperature (hereinafter, that may be referred to as inlet temperature of the heat medium) of the reactor for the heat medium for the adjustment of reaction temperature in the present invention is the highest temperature among them.

To be more specific, in the case of three-layer filling, when each reaction peak temperature in terms of mean value of each reaction tube is 360°C for the first layer, 370°C for the second layer and 350°C for the third layer and the heat medium inlet temperature is 330°C, the temperature difference of 40°C between 370°C for the second layer and 330°C for the heat medium inlet temperature is a value for the judgment whether the process of the present invention is able to be applied effectively. In the process of the present invention, an effective application is possible when "the temperature difference is 20°C or higher" and, in the case of the above-mentioned example, the process of the present invention is effectively applicable.

In case an operation is carried out when the temperature difference between the highest reaction peak temperature and the heat medium inlet temperature is less than 20°C, changes in the highest reaction peak temperature to the heat medium inlet temperature is small (even when the heat medium inlet temperature is changed to an extent of 1°C, change in the highest reaction peak temperature is about 1 to 2°C) and, therefore, the process of the present invention may be applied.

In the present invention, there is no particular limitation for the method for settling the position showing the reaction peak temperature in the direction of tube axis of each catalyst layer and its examples are the means where ratio of the inert substance to the catalyst, shape of the catalyst, type of the catalyst (composition, burning temperature upon preparation of the catalyst, etc.) and the like are appropriately modified. In the case of a carried catalyst, a means where carried amount of the catalytically active component is changed may be adopted as well.

### Adjustment of the inlet temperature for the heat medium

In a multi-tubular reactor used for a gas-phase catalytic oxidation of propylene and the like, there are several thousand to several tens thousand reaction tubes and, in the conventional filling methods, it is very difficult that the state of filling of the catalyst in all of the reaction tubes is made uniform.

A specific example is that, due to the difference in pressure loss generated by the difference in the filling operation, amount of gas flown into the reaction tube changes and state of the reaction changes for each reaction tube whereby, even in the same reactor, there is resulted a state where the reaction state differs for each reaction tube.

Further, reaction temperature is decided by a mean value of the reaction state for all reaction tubes. For example, in a former stage reactor with an object of oxidation reaction of propylene, there is dispersion in the convertion rates of propylene for each reaction tube and, therefore, the heat medium inlet temperature is decided by a mean value of the propylene convertion rates of all reaction tubes. Accordingly, it is not true that all reaction tubes are always operated under the optimum condition.

Under such circumstances, when the operation condition is constant, there may be no problem but, under a non-stationary state such as the case where supplying amount of raw material gas is changed due to the adjustment of production or the like, inactivation of the catalyst is resulted by, for example, formation of hot spot due to inconvenience such as that, as mentioned above, state of reaction is different in each reaction tube or all reaction tubes are not operated under the optimum condition.

As a means for avoiding the above, the present invention adopts a condition for changing the heat medium inlet temperature specified by the present invention whereby such inconveniences are solved.

To be more specific, in an operation of changing the heat medium inlet temperature,
(1) the change is carried out with the range of 2 °C or less per one operation of the change; and
(2) one operation of the change is carried out with a time interval of 10 minutes or more from the other operation of the change which is carried out just prior to the one operation of the change if one operation of the change is carried out successively to the other operation of the change.

When the changing operation is more than 2°C, the non-stationary state in the change promotes the reaction peak temperature whereby inactivation of the catalyst is apt to take place.

Further, when the changing operations are conducted successively, changes in the system by the changing operation are unable to follow the changing operation and, again, it promotes the peak temperature whereby inactivation of the catalyst is apt to take place. As a result of the investigation by the present inventors, the time interval for the changing operations is set not shorter than 10 minutes or, preferably, not shorter than 20 minutes.

### Method for adjusting the heat medium inlet temperature

In the present invention, any method may be used as a method for the adjustment of inlet temperature for the heat medium.

For example, a heat exchanger is set at 8b of Fig. 1, heat is removed from a part of or all of the heat medium followed by returning to the reactor from 8a whereupon the heat medium temperature is able to be adjusted. There is no particular limitation for the type of the above heat exchanger. To be more specific, a longitudinal fixed tube plate type, a transverse fixed tube plate type, a U-shaped tube type, a double tube type, spiral type, a square block type, etc. may be listed. With regard to the material, the frequently used ones are carbon steel,stainless steel, etc. but they are not limitative. Selection may be done from the viewpoint of heat resistance, corrosion resistance, economy, etc.

As hereunder, additional matters for the present invention will be mentioned.

### Step for the production of acrylic acid or acrylate

With regard to the step for the production of acrylic acid using an oxidation reaction control using the present invention as mentioned above, the following (i) to (iii) may, for example, be listed:
(i) an oxidation step where propylene and optionally propane and/or acrolein are/is subjected to a catalytic gas-phase oxidation, a catching step where acrylic acid-containing gas from the oxidation step is contacted with water so that acrylic acid is caught as an aqueous solution of acrylic acid and an extraction step where acrylic acid is extracted from the aqueous solution of acrylic acid using an appropriate extracting solvent are done, then acrylic acid is separated from the solvent, purification is conducted by a purification step and, further, a Michael adduct of acrylic acid and a high-boiling liquid containing a polymerization inhibitor used in each step are supplied to a degradation reaction tower as raw materials to recover valuables and the valuables are supplied to any of the steps after the catching step;
(ii) an oxidation step where propylene and optionally propane and/or acrolein are/is subjected to a catalytic gas-phase oxidation to produce acrylic acid, a catching step where acrylic acid-containing gas from the oxidation step is contacted with water so that acrylic acid is caught as an aqueous solution of acrylic acid, an azeotropic separation step where the aqueous solution of acrylic acid is distilled in an azeotropic separation tower in the presence of an azeotropic solvent whereupon crude acrylic acid is taken out from the bottom of the tower and a step for separation of acetic acid where acetic acid is removed and a purification for high-boiling impurities are done, then a Michael adduct of acrylic acid after the purification and a high-boiling liquid containing a polymerization inhibitor used for those production steps are supplied to a degradation reaction tower as raw materials to recover the valuables and the valuables are supplied to any of the steps after the catching step; and
(iii) an oxidation step where acrylic acid is produced by a catalytic gas-phase oxidation of propylene and optionally propane and/or acrolein, a catching/separation step where acrylic acid-containing gas is contacted with an organic solvent to catch acrylic acid as a solution of acrylic acid in the organic solvent whereby water, acetic acid, etc. are removed at the same time, a separation step where acrylic acid is taken out from the solution of acrylic acid in the organic solvent, a step where a high-boiling liquid containing a Michael adduct of acrylic acid, organic solvent and polymerization inhibitor used in those production steps is supplied to a degradation reaction tower as a raw material to recover the valuables and the valuables are supplied to any of the steps after the catching step and a step where the organic solvent is partially purified are done.

A step for the production of acrylate comprises an esterification reaction step where, for example, acrylic acid is made to react with alcohol using an organic acid or a cationic ion-exchange resin as a catalyst and a purification step where extraction, evaporation and distillation are conducted as unit operations for concentrating the crude acrylate prepared in the reaction. Each unit operation is appropriately selected by the raw material ratio of acrylic acid to alcohol in the esterification reaction, catalyst species used for the esterification reaction or physical properties of each of raw materials, by-products of the reaction and acrylate. After each of the unit operations, the product is prepared by a purification tower for acrylate. The liquid from the bottom of the purification tower contains Michael adducts mainly comprising acrylate, β-acryloxypropionate, β-alkoxypropionate and β-hydroxypropionate and is further supplied to a degradation reaction tower as a high-boiling liquid containing a polymerization inhibitor used for the production step or returned to a process whereby valuables are recovered.

In the production of acrylic acid or acrylate which is an easily-polymerizing compound, a polymerization inhibitor is used for the suppression of generation of polymers during the production.

Specific examples of the polymerization inhibitor are copper acrylate, copper dithiocarbamate, phenol compound and phenothiazine compound. Examples of the copper dithiocarbamate are copper dialkyldithiocarbamate such as copper dimethyldithiocarbamate, copper diethyldithiocarbamate, copper dipropyldithiocarbamate and copper dibutyldithiocarbamate; copper cycloalkylene-dithiocarbamate such as copper ethylenedithiocarbamate copper tetramethylenedithiocarbamate, copper penta-methylenedithiocarbamate and copper hexamethylene-dithiocarbamate; and copper cyclic oxydialkylene-dithiocarbamate such as copper oxydiethylene-dithiocarbamate. Examples of the phenol compound are hydroquinone, methoquinone, pyrogallol, catechol, resorcinol, phenol and cresol. Examples of the phenothiazine are phenothiazine, bis(α-methylbenzyl)-phenothiazine, 3,7-dioctylphenothiazine and bis(α-dimethylbenzyl)phenothiazine.

Substances other than the above-mentioned ones may be also included in some processes and it is apparent that the type thereof does not affect the present invention.

Acrylic acid or acrylate produced as such is used for various uses. Specific examples of the uses are superabsorbent polymer, flocculant, pressure-sensitive adhesive, paint, adhesive and fiber reforming agent.

### Examples

The present invention will now be specifically illustrated by way of the following Examples and Comparative Examples but is not limited thereto.

### Example 1

### Catalyst

Ammonium paramolybdate (94 parts by weight) was dissolved in 400 parts by weight of pure water with heating. On the other hand, 7.2 parts by weight of ferric nitrate, 25 parts by weight of cobalt nitrate and 38 parts by weight of nickel nitrate were dissolved in 60 parts by weight of pure water with heating. Those solutions were mixed with sufficient stirring to give a solution in a slurry form.

After that, 0.85 parts by weight of borax and 0.36 parts by weight of potassium nitrate were dissolved in 40 parts by weight of pure water with heating and added to the above slurry. Then 64 parts by weight of granular silica was added thereto followed by stirring. After that, 58 parts by weight of bismuth subcarbonate previously compounded with 0.8 % by weight of Mg was added thereto followed by stirring/mixing, the slurry was dried by heating and subjected to a heating treatment in an air atmosphere at 300°C for 1 hour and the resulting granular solid was made into tablets each having 5 mm diameter and 4 mm height by means of tablet compression using a molding machine and burned at 500°C for 4 hours to give a former stage catalyst.

The resulting former stage catalyst was a compounded oxide of an Mo-Bi type having a composition ratio of catalyst powder of a composition of Mo (12) Bi (5) Ni (3) Co (2) Fe (0.4) Na (0.2) Mg (0.4) B (0.2) K (0.1) Si (24) O (x) (where x which is a composition for oxygen is a value decided by the oxidized state of each metal).

### Production of acrylic acid and acrolein from propylene

In this example, a multi-tubular reactor which was the same as that shown in Fig. 1 was used.

To be more specific, a multi-tubular reactor of a reactor shell (inner diameter: 4,500 mm) having 10,000 reaction tubes made of stainless steel where each reaction tube had 3.5 m length and 27 mm inner diameter was used. The reaction tube was not placed at the circular opening region at the center of disk-shaped baffle plate 6a having opening near the center of the reactor shell. In the baffle plate, a perforated disk-shaped baffle plate 6a having an opening near the center of the reactor shell and a perforated disk-shaped baffle plate 6b arranged so as to have an opening between the outer circumference and the reactor shell were arranged in the order of 6a-6b-6a with same intervals wherein the opening ratio of each of the baffle plates was 18%.

A fused salt of nitrate mixture (a niter) was used as a heat medium and it was supplied from the lower part of the reactor and taken out from the upper part of the reactor to circulate.

A part of this heat medium was taken out from 8b to remove the heat and was returned to 8a. As a result, temperature of the heat medium supplied to the reactor was adjusted and the temperature was measured by a thermometer

With regard to a catalyst to be filled in each reaction tube, that where catalytic activity was adjusted by mixing of the above-mentioned former stage catalyst and balls made of silica each having 5 mm diameter and having no catalytic activity was used and filled from the inlet of the reaction tube so as to make the ratio of catalytic activity 0.5, 0.7 and 1 to form a three-layered catalyst layer.

The raw material gas was supplied from the upper part of the reactor so as to make it a counter-current type to the heat medium and a raw material gas comprising 9 molar % concentration of propylene, 14.5 molar % concentration of molecular oxygen, 9 molar % of water and 67.5 molar % of nitrogen of 75 kPa (gauge pressure) was supplied at 12,300 Nm³/hour. A thermometer having ten measuring points in the direction of tube axis was inserted into the reaction tube to measure the temperature distribution.

When temperature of inlet for the heat medium (temperature of inlet for the niter) was set at 335°C and an operation was conducted for one week, the reaction peak temperature of the first layer catalyst was highest showing 395°C and convertion rate of propylene was 97% while the total yield of acrolein and acrylic acid was 92%. As to the reaction temperature, temperature of the niter which was supplied to the reactor was used. Temperature difference between inlet and outlet for the niter was 5°C.

The raw material gas was increased to make 13,530 Nm³/hour (an increase of 10%).

In order to give the same convertion rate (97%) of propylene, temperature of inlet for the heat medium was raised to an extent of 1°C, then raised again to an extent of 1°C after 1 hour and, after 1 hour more, raised to an extent of 1°C to give 338°C.

After 2 hours, the reaction gas showed a convertion rate of 97% in terms of propylene and the total yield for acrolein and acrylic acid was 92%. The highest reaction peak temperature of the first catalyst layer showed 405°C and a stable operation continued.

### Comparative Example 1

An operation was carried out by the same method as in Example 1 except that method for changing the inlet temperature for the heat medium was changed as follows.

Inlet temperature for the heat medium was raised to an extent of 3°C from 335°C in one operation to give 338°C.

After a short time, the highest reaction peak temperature of the first catalyst layer rose to 440°C whereupon the reaction gas showed a convertion rate of 99% in terms of propylene and the total yield of acrolein and acrylic acid was 89%. Accordingly, the inlet temperature for the heat medium was lowered to an extent of 2°C to give 336°C.

### Comparative Example 2

An operation was carried out by the same method as in Example 1 except that method for changing the inlet temperature for the heat medium was changed as follows.

Inlet temperature for the heat medium was raised to an extent of 1°C from 335°C, raised to an extent of 1°C after 5 minutes and, after 5 minutes more, raised to an extent of 1°C to give 338°C.

After a short time, the highest reaction peak temperature of the first catalyst layer rose to 436°C whereupon the reaction gas showed a convertion rate of 98.8% in terms of propylene and the total yield of acrolein and acrylic acid was 89.3%. Accordingly, the inlet temperature for the heat medium was lowered to an extent of 2°C to give 336°C.

### Example 2

An operation was carried out under the same condition as in Example 1 except that the temperature difference of the niter to be supplied to the reactor between inlet and outlet was made 3°C.

When an operation was conducted for one week after setting the inlet temperature for the heat medium 337°C, the reaction peak temperature of the first catalyst layer was highest showing 390°C. Convertion rate of propylene was 97% and the total yield of acrolein and acrylic acid was 92%.

The raw material gas was increased to 13,530 Nm³/hour (an increase of 10%).

In order to achieve the same convertion rate of propylene of 97%, the inlet temperature of the heat medium was raised to an extent of 1°C, further raised to an extent of 1°C after 1 hour and, after 1 hour more, raised to an extent of 1°C to give 340°C.

After 2 hours, convertion rate of the reaction gas in terms of propylene was 97.5% while the total yield of acrolein and acrylic acid was 93% and the highest reaction peak temperature of the first catalyst layer was 400°C whereupon a stable operation was able to be continued.

### Comparative Example 3

An operation was carried out by the same method as in Example 2 except that method for changing the inlet temperature for the heat medium was changed as follows.

Inlet temperature for the heat medium was raised to an extent of 3°C from 337°C in an operation to give 340°C.

After a short time, the highest reaction peak temperature of the first catalyst layer rose to 438°C whereupon the reaction gas showed a convertion rate of 99% in terms of propylene and the total yield of acrolein and acrylic acid was 89%. Accordingly, the inlet temperature for the heat medium was lowered to an extent of 2°C to give 338°C.

### Example 3

### Catalyst

As to a catalyst for a gas-phase catalytic oxidation of propylene, a catalyst having the following composition (in atomic ratio) was prepared by a method disclosed in the JP-A-63-54942.

Mo : Bi : Co : Fe : Na : B : K : Si : O = 12 : 1 : 0.6 : 7 : 0.1 : 0.2 : 0.1 : 18 : X (in which X which is a composition of oxygen is a value decided by the oxidized state of each metal element.)

Into the reaction tube were filled 0.43 L of a mixture of 50% catalyst and 30% alumina balls in terms of ratio by volume as the first layer, 0.43 L of a mixture of 70% catalyst and 30% alumina balls in terms of ratio by volume as the second layer and 0.86 L of the catalyst as the third layer.

### Reaction method

Reaction was carried out in the same reactor as Example 1 under the condition of the raw material gas composition of Example 1 where the supplying amount was 10,320 Nm³/hour.

When the inlet temperature for the heat medium was made 323°C, the reaction peak temperature of the first catalyst layer was highest showing 380°C and the convertion rate of propylene was 97% while the total yield of acrylic acid and acrolein was 92%.

After that, the raw material gas was increased to make 11,868 Nm³/hour (an increase of 15%).

In order to achieve the same convertion rate of propylene of 97%, the inlet temperature of the heat medium was raised to an extent of 2°C, further raised to an extent of 1°C after 1 hour and, after 1 hour more, raised to an extent of 1°C to give 327°C.

After 2 hours, the reaction gas showed a convertion rate of propylene of 97%, the total yield of acrolein and acrylic acid was 92% and the highest reaction peak temperature of the first catalyst layer was 395°C whereupon a stable operation was able to be continued.

### Comparative Example 4

An operation was carried out by the same method as in Example 3 except that method for changing the inlet temperature for the heat medium was changed as follows.

Inlet temperature for the heat medium was raised to an extent of 4°C from 323°C in an operation to give 327°C.

After a short time, the highest reaction peak temperature of the first catalyst layer rose to 445°C and, accordingly, an emergency stop operation was conducted for the protection of the catalyst.

### Comparative Example 5

An operation was carried out by the same method as in Example 3 except that method for changing the inlet temperature for the heat medium was changed as follows.

Inlet temperature for the heat medium was raised to an extent of 2°C from 323°C, raised to an extent of 1°C after 5 minutes and, after 5 minutes more, raised to an extent of 1°C to give 327°C.

After a short time, the highest reaction peak temperature of the first catalyst layer rose to 440°C whereupon the reaction gas showed a convertion rate of 99% in terms of propylene and the total yield of acrolein and acrylic acid was 88%. Accordingly, the inlet temperature for the heat medium was lowered to an extent of 2°C to make 325°C and that state was maintained.

After a short time, the reaction peak temperature lowered reaching 376°C. Since convertion rate of propylene became 95%, the method for changing the inlet temperature for the heat medium of Example 3 was conducted so as to raise to 97%, whereupon stable state could be obtained.

While the invention has been described in detail and with reference to specific embodiments thereof, it will be apparent to one skilled in the art that various changes and modifications can be made therein without departing from the scope of the claims.

This application is based on the Japanese patent application (Application No. 2003-416718) filed on December 15, 2003.

### Industrial Applicability

In a process for the production of acrolein or acrylic acid where a gas-phase catalytic oxidation reaction is carried out for the production raw material for acrolein or acrylic acid with molecular oxygen or molecular oxygen-containing gas using a multi-tubular reactor equipped with one or more catalyst layer(s) in a direction of tube axis, change in the inlet temperature of heat medium for the adjustment of reaction temperature is conducted by a method specified as mentioned above for enhancing the production velocity whereby it is now possible to conduct the change in the temperature condition in a stable manner without a sudden rise of temperature and, as a result, it is possible to change the production condition for enhancing the production velocity without deterioration of the catalyst and a stable and highly efficient production is able to be continued.

## Claims

1. A process for the production of acrylic acid or acrolein by a gas-phase catalytic oxidation reaction of propylene with molecular oxygen or gas which contains molecular oxygen using a multi-tubular reactor having such a structure that there are plural reaction tubes equipped with one or more Mo-Bi type catalyst layer(s) in a direction of tube axis and a heat medium for the adjustment of reaction temperature is able to flow outside those reaction tubes; wherein the process is **characterized in that** the change for raising the reaction temperature of said gas-phase catalytic oxidation reaction is conducted by means of change in inlet temperature of the heat medium for the adjustment of reaction temperature together with (1) the change in the inlet temperature of the heat medium for the adjustment of the reaction temperature is carried out with the range of 2 °C or less per one operation of the change; and (2) one operation of the change is carried out with a time interval of 10 minutes or more from the other operation of the change which is carried out just prior to the one operation of the change if one operation of the change is carried out successively to the other operation of the change.

2. The process according to claim 1, wherein the difference between the maximum value of the reaction peak temperature of the catalyst layer of the reaction tube and the inlet temperature of the heat medium for adjustment of the reaction temperature is not lower than 20 °C.

3. The process according to claim 1 or 2, wherein activity of each catalyst layer of the reaction tube is adjusted by mixing of an inert substance.

4. The process according to claim 1 or 2, wherein the number of the catalyst layers of the reaction tube is from 1 to 10.

5. The process according to claim 3, wherein the number of the catalyst layers of the reaction tube is from 1 to 10.

## Patentansprüche

1. Ein Verfahren zur Herstellung von Acrylsäure oder Acrolein durch eine katalytische Gasphasen-Oxidationsreaktion von Propylen mit molekularem Sauerstoff oder Gas, welches molekularen Sauerstoff enthält, unter Verwendung eines Mehrrohrreaktors, der einen derartigen Aufbau aufweist, bei dem mehrere Reaktionsrohre vorliegen, die mit einer oder mehreren Katalysatorschicht(en) vom Mo-Bi-Typ, die in eine Richtung der Rohrachse vorliegen, ausgestattet sind, und bei dem ein Wärmemedium zur Einstellung der Reaktionstemperatur außerhalb dieser Reaktionsrohre fließen kann; wobei das Verfahren **dadurch gekennzeichnet ist, dass** die Änderung zur Erhöhung der Reaktionstemperatur der katalytischen Gasphasen-Oxidationsreaktion durchgeführt wird, durch eine Änderung der Eintrittstemperatur des Wärmemediums zur Einstellung der Reaktionstemperatur zusammen mit (1) die Änderung der Eintrittstemperatur des Wärmemediums zur Einstellung der Reaktionstemperatur wird durchgeführt mit dem Bereich von 2 °C oder weniger pro Änderungsvorgang; und (2) ein Änderungsvorgang wird durchgeführt mit einem Zeitabstand von 10 Minuten oder mehr von dem anderen Änderungsvorgang, welcher gerade vor dem Änderungsvorgang durchgeführt wird, wenn ein Änderungsvorgang im Anschluß an den anderen Änderungsvorgang durchgeführt wird.

2. Das Verfahren gemäß Anspruch 1, wobei der Unterschied zwischen dem Maximalwert der Reaktionshöchsttemperatur der Katalysatorschicht des Reaktionsrohres und der Eintrittstemperatur des Wärmemediums zur Einstellung der Reaktionstemperatur nicht weniger als 20°C beträgt.

3. Das Verfahren gemäß Anspruch 1 oder 2, wobei die Aktivität jeder Katalysatorschicht des Reaktionsrohres durch Mischen mit einer inerten Substanz eingestellt wird.

4. Das Verfahren gemäß Anspruch 1 oder 2, wobei die Anzahl der Katalysatorschichten des Reaktionsrohres 1 bis 10 beträgt.

5. Das Verfahren gemäß Anspruch 3, wobei die Anzahl der Katalysatorschichten des Reaktionsrohres 1 bis 10 beträgt.

## Revendications

1. Procédé pour la production d'acide acrylique ou d'acroléine via une mise en réaction d'oxydation catalytique en phase gazeuse de propylène avec de l'oxygène moléculaire ou un gaz qui contient de l'oxygène moléculaire en utilisant un réacteur multitubulaire possédant une structure telle que plusieurs tubes réactionnels sont munis d'une ou de plusieurs couches de catalyseur de type Mo-Bi dans une direction de l'axe du tube, un milieu chauffant pour le réglage de la température réactionnelle étant à même de s'écouler à l'extérieur de ces tubes réactionnels, le procédé étant **caractérisé en ce que** le changement pour l'élévation de la température réactionnelle de ladite réaction d'oxydation catalytique en phase gazeuse est mis en oeuvre au moyen d'un chargement de la température d'entrée du milieu de chauffage pour le réglage de la température réactionnelle, conjointement avec (1) le fait que le changement de la température d'entrée du milieu de chauffage pour le réglage de la température réactionnelle est réalisé avec la gamme de 2 °C ou moins pour une opération de changement ; et (2) une opération de changement est mise en oeuvre avec un intervalle de temps de 10 minutes ou plus à partir de l'autre opération de changement qui est mise en oeuvre directement avant ladite une opération de changement si une opération de changement est mise en oeuvre successivement suivante l'autre opération de changement.

2. Procédé selon la revendication 1, dans lequel la différence entre la valeur maximale de la température réactionnelle maximale de la couche de catalyseur du tube réactionnel et la température d'entrée du milieu chauffant pour le réglage de la température réactionnelle n'est pas inférieure à 20 °C.

3. Procédé selon la revendication 1 ou 2, dans lequel l'activité de chaque couche de catalyseur du tube réactionnel est réglée par mélange avec une substance inerte.

4. Procédé selon la revendication 1 ou 2, dans lequel le nombre des couches de catalyseur du tube réactionnel s'élève de 1 à 10.

5. Procédé selon la revendication 3, dans lequel le nombre des couches de catalyseur du tube réactionnel s'élève de 1 à 10.
